(19) Europäisches Patentamt
European Patent Office
Office européen des brevets

(11) EP 1 182 203 A1

(12) **EUROPEAN PATENT APPLICATION**
published in accordance with Art. 158(3) EPC

(43) Date of publication:
**27.02.2002 Bulletin 2002/09**

(21) Application number: **00913081.6**

(22) Date of filing: **03.04.2000**

(51) Int Cl.[7]: **C07D 409/08**, A01N 43/56

(86) International application number:
**PCT/JP00/02154**

(87) International publication number:
**WO 00/69853 (23.11.2000 Gazette 2000/47)**

(84) Designated Contracting States:
**AT BE CH CY DE DK ES FI FR GB GR IE IT LI LU MC NL PT SE**
Designated Extension States:
**AL LT LV MK RO SI**

(30) Priority: **13.05.1999 JP 13279199**

(71) Applicant: **Idemitsu Kosan Co., Ltd.**
**Tokyo 100-0005 (JP)**

(72) Inventors:
- **SAITOU, Masatoshi**
  **Sodegaura-shi Chiba-ken 299-0205 (JP)**
- **SEKIGUCHI, Hiroki**
  **Sodegaura-shi Chiba-ken 299-0205 (JP)**
- **OGAWA, Shinichiro**
  **Sodegaura-shi Chiba-ken 299-0205 (JP)**

(74) Representative:
**Gille Hrabal Struck Neidlein Prop Roos**
**Patentanwälte Brucknerstrasse 20**
**40593 Düsseldorf (DE)**

(54) **AZOLE COMPOUNDS AND HERBICIDE COMPOSITIONS**

(57) Azole compounds are represented by the following formula (Ia) or (Ib):

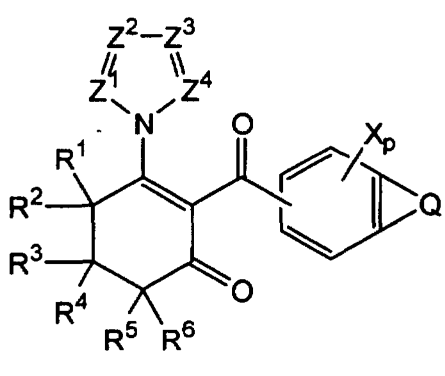

(Ia),

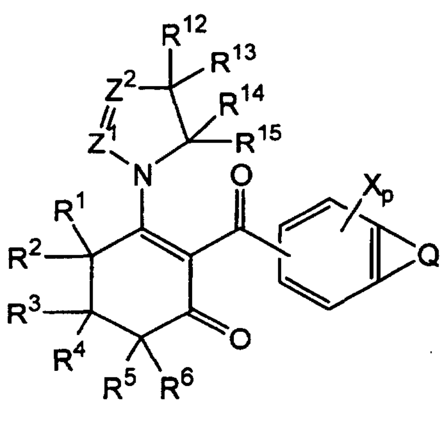

(Ib),

EP 1 182 203 A1

wherein, Q, X, p, $R^1$ to $R^6$, $R^{12}$ to $R^{15}$ and $Z^1$ to $Z^4$ are as defined in the disclosure, and herbicidal compositions contain the azole compounds of formula (Ia) or (Ib) in herbicidally effective amounts. The compounds of the formulae (Ia) and (Ib) are less injurious to cultivated crops and have a broad spectrum of weed control with low application rates.

## Description

### Technical Field

The present invention relates to novel azole compounds and herbicidal compositions containing the azole compounds as the effective components. More particularly, the present invention relates to azole compounds useful for controlling undesired cropland weeds and paddy weeds which are detrimental to cultivated plants, and herbicidal compositions containing the azole compounds as the effective components.

### Background Art

Heretofore, as compounds having chemical structures similar to those of azole compounds, heterocyclic compounds having a monocyclic benzoyl moiety and a saturated N-containing heterocyclic ring such as morpholine ring, pyrrolidine ring and piperidine ring have been known (Japanese Patent Application Laid-Open Nos. 62-298563 and 7-196585). However, no azole compounds having condensed ring benzoyl structures have heretofore been known. In the above prior arts, no descriptions can be found about azole compounds having a benzoyl moiety with condensed ring although it is taught that compounds having a monocyclic benzoyl moiety are herbicidally active.

Herbicides are chemicals important for saving the work of controlling weeds which are detrimental to growth of cultivated plants, and for achieving high productivity of agricultural and horticultural crops. Therefore, development of new herbicides which are safe for human being and livestock, less injurious to cultivated crops due to their excellent selective control of only weeds and broad spectrum of weed control have long been desired.

### Disclosure of Invention

An object of the present invention is to provide novel azole compounds which are less injurious to cultivated crops, particularly to corn, wheat and paddy rice and show broad spectrum of weed control even in low application rates. Another object of the present invention is to provide herbicidal compositions containing the azole compounds as the effective components.

As the result of extensive studies by the present inventors to achieve the above objects, it has been found that azole compounds having specific structures are less injurious to cultivated crops such as corn, wheat and paddy rice and highly effective for controlling various weed species with low application rates. The present invention has been completed based on this finding.

Accordingly, the present invention is directed to:

(1) An azole compound represented by the following formula (Ia) or (Ib):

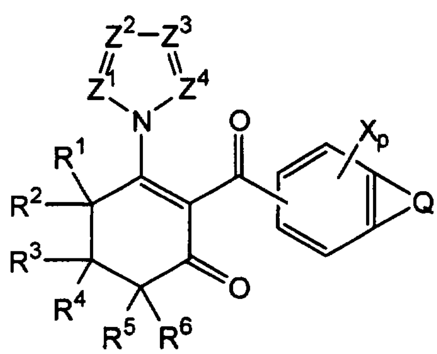 (Ia),

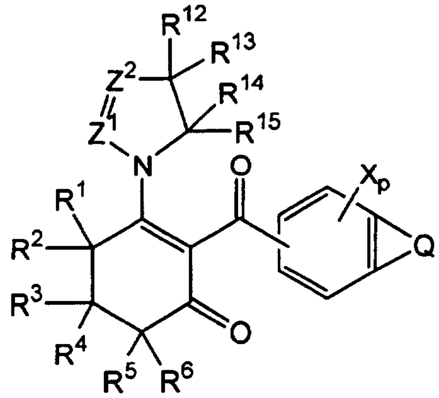

(Ib)

wherein, Q is a divalent group having 3 to 5 ring-forming atoms which form a five- to seven-membered saturated or unsaturated condensed ring together with two benzene ring carbons to which Q is bonded, one or two of the ring-forming atoms being atom or atoms selected from nitrogen, oxygen and sulfur; the ring-forming atoms constituting Q may have one or more substituents selected from the group consisting of $C_1$-$C_6$ alkyl, $C_1$-$C_6$ haloalkyl, $C_1$-$C_6$ alkoxy, $C_1$-$C_6$ haloalkoxy, hydroxyl, mercapto, oxo, thioxo, hydroxyimino, $C_1$-$C_6$ alkoxyimino, hydrazono, $C_1$-$C_6$ monoalkylhydrazono and $C_1$-$C_6$ dialkylhydrazono; the ring-forming atom constituting Q or a pair of adjacent ring-forming atoms may be substituted by a divalent substituent selected from the group consisting of ethylenedioxy, ethylenedithio, propylenedioxy and propylenedithio, the divalent substituent being optionally substituted by halogen or $C_1$-$C_6$ alkyl; X is halogen, $C_1$-$C_6$ alkyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, amino, $C_1$-$C_6$ monoalkylamino, $C_1$-$C_6$ dialkylamino, cyano or nitro; p is 1 or 2; $R^1$ to $R^6$ are each hydrogen, halogen, $C_1$-$C_6$ alkyl or $C_1$-$C_6$ haloalkyl; $R^{12}$ to $R^{15}$ are each hydrogen, halogen, $C_1$-$C_6$ alkyl, $C_1$-$C_6$ haloalkyl, nitro or phenyl which may be substituted; and $Z^1$ to $Z^4$ are each nitrogen or $CR^7$ wherein $R^7$ is hydrogen, halogen, $C_1$-$C_6$ alkyl, $C_1$-$C_6$ haloalkyl, nitro or phenyl which may be substituted, and a pair of adjacent $CR^7$, if any, may together form a benzene ring which may be substituted by halogen, $C_1$-$C_6$ alkyl, $C_1$-$C_6$ haloalkyl or nitro;

(2) An azole compound described in (1), wherein $Z^1$ of the formula (Ia) or (Ib) is nitrogen;

(3) An azole compound described in (1), wherein at least one of two ring-forming atoms constituting Q of the formula (Ia) or (Ib) bonded to the benzene ring is sulfur;

(4) An azole compound described in (1), wherein $Z^1$ of the formula (Ia) or (Ib) is nitrogen and at least one of two ring-forming atoms constituting Q of the formula (Ia) or (Ib) bonded to the benzene ring is sulfur; and

(5) A herbicidal composition containing an effective amount of an azole compound described in any of (1) to (4).

## Best Mode for Carrying Out the Invention

The azole compounds of the present invention have the chemical structures represented by the formula (Ia) or (Ib) described above.

In the formula (Ia) or (Ib), Q is a divalent group having 3 to 5 ring-forming atoms which form a five- to seven-membered saturated or unsaturated condensed ring together with two benzene ring carbons to which Q is bonded. One or two of the ring-forming atoms are atoms selected from nitrogen, oxygen and sulfur, and the other of the ring-forming atoms is carbon. The ring-forming sulfur may form -SO- or $-SO_2-$. The ring-forming atoms constituting Q may have one or more substituents selected from the group consisting of $C_1$-$C_6$ alkyl, $C_1$-$C_6$ haloalkyl, $C_1$-$C_6$ alkoxy, $C_1$-$C_6$ haloalkoxy, hydroxyl, mercapto, oxo, thioxo, hydroxyimino, $C_1$-$C_6$ alkoxyimino, hydrazono, $C_1$-$C_6$ monoalkylhydrazono and $C_1$-$C_6$ dialkylhydrazono. The ring-forming atom of Q or a pair of adjacent ring-forming atoms may be substituted by a divalent substituent selected from the group consisting of ethylenedioxy, ethylenedithio, propylenedioxy and propylenedithio. The divalent substituent being optionally substituted by halogen or $C_1$-$C_6$ alkyl.

$C_1$-$C_6$ Alkyl may include methyl, ethyl and different isomers of propyl, butyl, pentyl and hexyl. Different isomers of propyl, butyl, pentyl and hexyl include linear, branched or cyclic groups, and these may contain an unsaturated bond. $C_1$-$C_6$ Haloalkyl may be haloalkyl obtained by partially or fully replacing hydrogen atoms of the above alkyls with halogen such as chlorine, fluorine, bromine and iodine. Specific examples of the haloalkyl include chloromethyl, difluoromethyl, trichloromethyl, trifluoromethyl, 2-chloroethyl, 2-fluoroethyl, 3-chloropropyl and 3-fluoropropyl.

$C_1$-$C_6$ Alkoxy may include methoxy, ethoxy and different isomers of propoxy, butoxy, pentyloxy and hexyloxy. Different isomers of propyloxy, butyloxy, pentyloxy and hexyloxy include linear, branched and cyclic groups, and these may contain an unsaturated bond. $C_1$-$C_6$ Haloalkoxy may include chloromethyloxy, difluoromethyloxy, trichloromethyloxy, trifluoromethyloxy, 2-chloroethyloxy, 2-fluoroethyloxy, 3-chloropropyloxy and 3-fluoropropyloxy. $C_1$-$C_6$ Alkoxyimino may include methoxyimino, ethoxyimino and different isomes of propoxyimino, butoxyimino, pentyloxyimino and hexyloxyimino. $C_1$-$C_6$ Monoalkylhydrazono may include methylhydrazono, ethylhydrazono and various isomers of propylhydrazono, butylhydrazono, pentylhydrazono and hexylhydrazono. $C_1$-$C_6$ Dialkylhydrazono include dimethylhydrazono, methylethylhydrazono, diethylhydrazono, di-n-propylhydrazono and di-i-propylhydrazono.

Halogen for X in the formula (Ia) or (Ib) may be fluorine , chlorine , bromine or iodine. $C_1$-$C_6$ alkyl, $C_1$-$C_6$ haloalkyl, $C_1$-$C_6$ alkoxy and $C_1$-$C_6$ haloalkoxy for X are respectively the same as those for Q. $C_1$-$C_6$ Alkylthio may include alkylthio such as methylthio, ethylthio and different isomers of propylthio, butylthio, pentylthio and hexylthio. Different isomers of propylthio, butylthio, pentylthio and hexylthio may be linear, branched or cyclic groups, and these may contain an unsaturated bond. $C_1$-$C_6$ alkylsulfinyl may include methylsulfinyl, ethylsulfinyl and different isomers of propylsufinyl, butylsulfinyl, pentylsulfinyl and hexylsulfinyl. $C_1$-$C_6$ alkylsulfonyl may include methylsulfonyl, ethylsulfonyl and different isomers of propylsulfonyl, butylsulfonyl, pentylsulfonyl and hexylsulfonyl. $C_1$-$C_6$ monoalkylamino may include methylamino, ethylamino and different isomers of propylamino, butylamino, pentylamino and hexylamino. $C_1$-$C_6$ dialkylamino may include dimethylamino, methylethylamino, diethylamino, di-n-propylamino and di-i-propylamino.

$C_1$-$C_6$ alkyl and $C_1$-$C_6$ haloalkyl for $R^1$ to $R^7$, $R^{12}$ to $R^{15}$ and the substituent on the benzene ring which may be formed by a pair of adjacent $CR^7$, are the same as those for Q. Phenyl, which may have a substituent, represented by $R^7$ or any of $R^{12}$ to $R^{15}$ may include phenyl, tolyl, m-chlorophenyl, p-methoxyphenyl, p-nitrophenyl and p-cyanophenyl.

In the formula (Ia) or (Ib), the azole structure represented by the following formula (II):

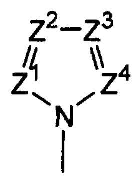

(II)

is preferably a residue derived form pyrrole, pyrazole, imidazole, triazole, tetrazole, indole, indazole and benzimidazole.

In the formula (Ia) or (Ib), the condensed ring structure represented by the following formula (III):

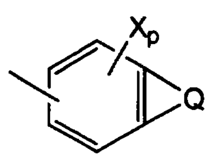

(III)

is preferably a residue derived from indene, benzofuran, benzothiophene, indole, naphthalene, chromene, thiochromene, quinoline, benzothiazole, benzisothiazole, benzoxazole, benzisoxazole, benzimidazole, indazole and saturated compounds thereof.

When $R^1$ and/or $R^6$ is hydrogen, the azole compounds represented by the formula (Ia) may be tautomerized as shown below:

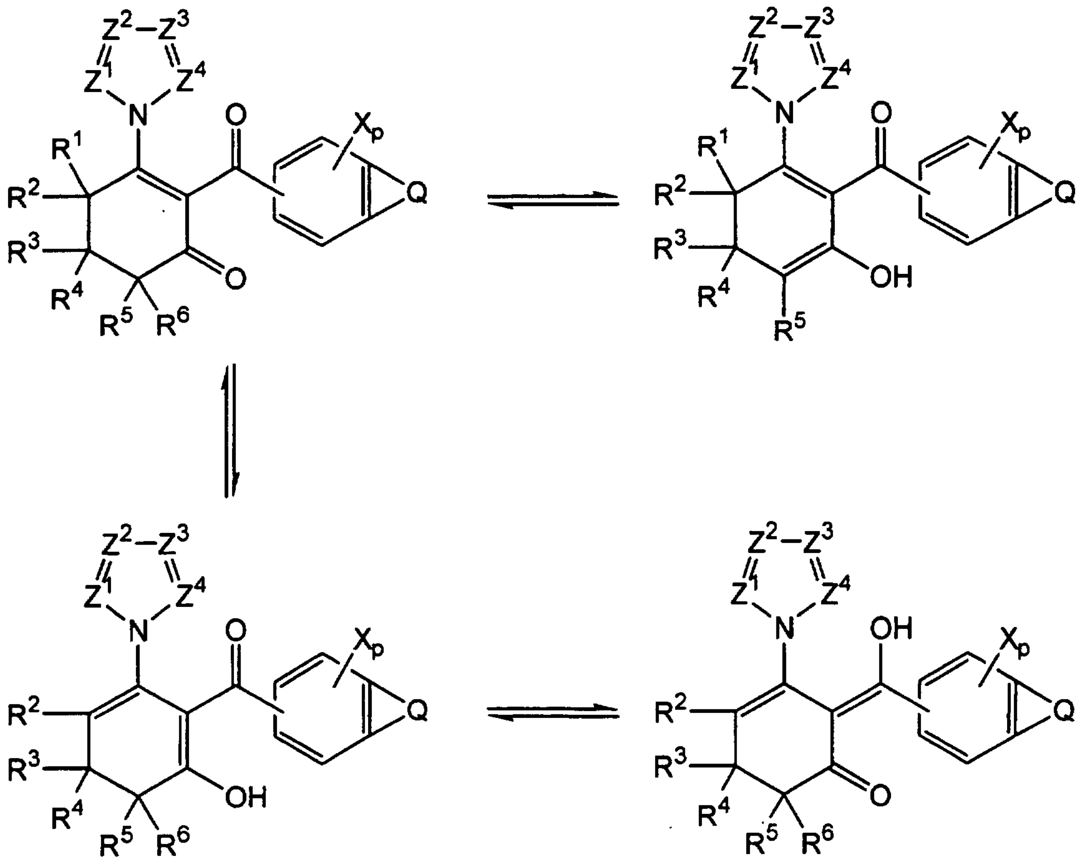

wherein $R^1$ to $R^6$, Q, X, $Z^1$ to $Z_4$ and p are the same as in the formula (Ia). The azole compounds of the formula (Ib) show the same tautomerism. The tautomers are included in the azole compounds of the present invention.

The azole compounds of the present invention may be also represented by the following formula (VIa) or (VIb):

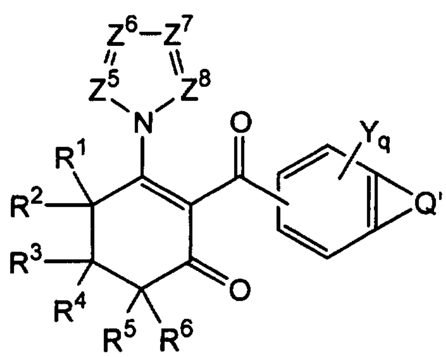

(VIa),

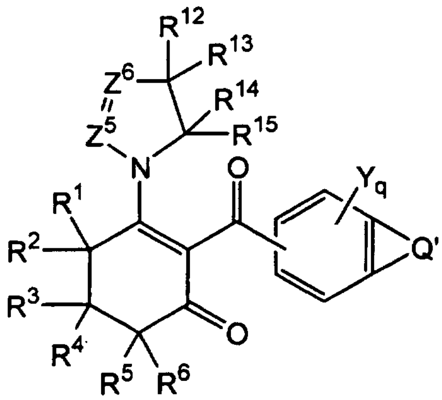

(VIb).

In the formula (VIa) or (VIb), $R^1$ to $R^6$ and $R^{12}$ to $R^{15}$ are as described above.

$Z^5$ to $Z^8$ are each nitrogen or $CR^{11}$ wherein $R^{11}$ is hydrogen, halogen, $C_1$-$C_6$ alkyl, $C_1$-$C_6$ haloalkyl, nitro, cyano or phenyl which may be substituted. When adjacent $Z^i$ and $Z^{i+1}$ wherein i is 5, 6 or 7 are both $CR^{11}$, the substituents on the carbon atoms may together form a benzene ring which may be substituted by halogen, $C_1$-$C_6$ alkyl, $C_1$-$C_6$ haloalkyl or nitro. $C_1$-$C_6$ alkyl and $C_1$-$C_6$ haloalkyl for $R^{11}$ and the substituent on the benzene ring, and the phenyl which may be substituted are the same as those for Q mentioned above.

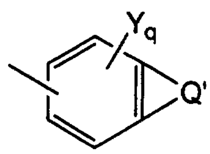

is 5 to 7-membered, satured or unsatured condensed ring represented by the following formula :

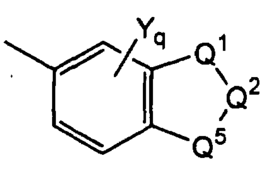
,
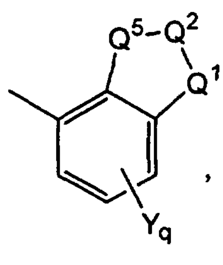
,
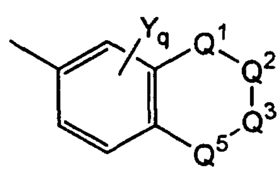
,
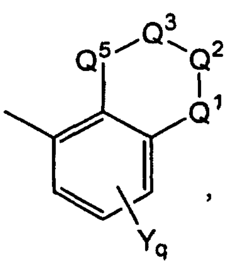
,
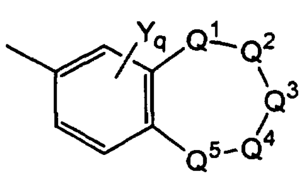
or
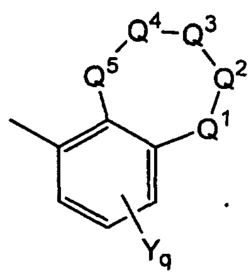
.

In the above formulae, up to two of the ring-forming atoms $Q^1$ to $Q^5$ are atoms selected from nitrogen, oxygen and sulfur, with the proviso that $Q^1$ is not sulfur. The ring-forming sulfur may form -SO- or -$SO_2$-. The ring-forming atoms $Q^1$ to $Q^5$ may have one or more substituents, which are the same as those for Q mentioned above, i.e., substituents selected from the group consisting of $C_1$-$C_6$ alkyl, $C_1$-$C_6$ haloalkyl, $C_1$-$C_6$ alkoxy, $C_1$-$C_6$ haloalkoxy, hydroxyl, mercapto, oxo, thioxo, hydroxyimino, $C_1$-$C_6$ alkoxyimino, hydrazono, $C_1$-$C_6$ monoalkylhydrazono and $C_1$-$C_6$ dialkylhy-

drazono. The ring-forming atom of Q' or a pair of adjacent ring-forming atoms may be substituted by a divalent substituent selected from the group consisting of ethylenedioxy, ethylenedithio, propylenedioxy and propylenedithio to form a ring structure which may be substituted by halogen or $C_1$-$C_6$ alkyl.

Y is halogen such as fluorine , chlorine , bromine and iodine, nitro, amino, cyano, hydroxy, mercapto, $R^8$, $OR^8$, $SR^8$, $SO_2R^8$, $NR^9R^{10}$ or $NHCOR^8$. $R^8$ is linear, branched or cyclic $C_1$-$C_6$ alkyl which may contain an unsaturated bond, linear, branched or cyclic $C_1$-$C_6$ haloalkyl which may contain an unsaturated bond, phenyl which may be substituted or benzyl which may be substituted. $R^9$ and $R^{10}$ are each hydrogen, linear, branched or cyclic $C_1$-$C_6$ alkyl which may contain an unsaturated bond, linear, branched or cyclic $C_1$-$C_6$ haloalkyl which may contain an unsaturated bond, phenyl which may be substituted or benzyl which may be substituted. $R^9$ and $R^{10}$ may be bonded to each other to form a ring structure. Specifically, $R^8$ is methyl, ethyl, different isomers of propyl, butyl, pentyl and hexyl, chloromethyl, difluoromethyl, trichloromethyl, trifluoromethyl, 2-chloroethyl, 2-fluoroethyl, 3-chloropropyl, 3-fluoropropyl, etc., and $NR^9R^{10}$ is methylamino, ethylamino, different isomers of propylamino, butylamino, pentylamino and hexylamino, dimethylamino, methylethylamino, diethylamino, di-n-propylamino, di-i-propylamino, etc.

The suffix "q" for Y is 0, 1 or 2.

The azole compounds of the present invention may be synthesized, for example, as shown below:

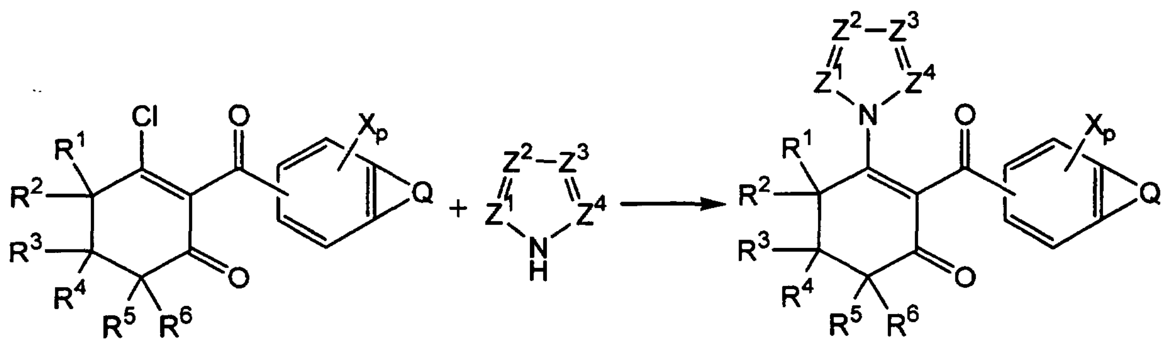

(IV) (V) (Ia)

wherein a monochlorinated compound of the formula (IV) is reacted with an azole of the formula (V) to obtain.

In this method, the azole compound is preferably made into salt form by an alkali metal compound such as sodium hydride prior to the reaction with the monochlorinated compound. An inert solvent such as tetrahydrofuran is preferably used as the solvent for the reaction. The reaction temperature may be from room temperature to the boiling point of the solvent, and stirring is preferably continued until the reaction is completed.

Alternatively, the above monochlorinated compound and an azole may be reacted without using solvent or in a solvent such as methylene chloride, 1,2-dichloroethane and chloroform at a temperature from room temperature to the boiling point of the solvent under stirring. In this reaction, an organic base such as triethylamine may be used as the catalyst.

The azole compounds represented by the formula (Ib), (VIa) or (VIb) may be synthesized in the same manner as mentioned above.

The herbicidal compositions of the present invention include the azole compounds represented by the formula (Ia), (Ib), (VIa) or (VIb) obtained in the manner described above as the effective components. The azole compounds may be used as a formulation with a liquid diluent such as a solvent or a solid diluent such as fine mineral powder. Useful formulations include wettable powders, emulsifiable concentrates, dusts and granules. The formulations may further contain a surfactant to make the compositions more emulsifiable, dispersible and spreadable.

Wettable powders preferably comprise 5 to 55% by weight of the azole compound, 40 to 93% by weight of a solid diluent and 2 to 5% by weight of a surfactant. Emulsifiable concentrates preferably comprise 10 to 50% by weight of the azole compound, 35 to 85% by weight of a solvent and 5 to 15% by weight of a surfactant. Dusts preferably comprise 1 to 15% by weight of the azole compound, 80 to 97% by weight of a solid diluent and 2 to 5% by weight of a surfactant. Granules may be prepared by granulating a composition preferably comprising 1 to 15% by weight of the azole compound, 80 to 97% by weight of a solid diluent and 2 to 5% by weight of a surfactant.

Preferred solid diluent may include oxides such as diatomaceous earth and slaked lime, phosphates such as apatite, sulfates such as gypsum and fine mineral powders such as talc, pyrophyllite, clay, kaolin, bentnite, acid clay, white carbon, quartz powder and silica powder.

Organic solvents are preferred for the solvent, and which may include aromatic hydrocarbons such as benzene, toluene and xylene; chlorinated hydrocarbons such as o-chlorotoluene, trichloroethane and trichloroethylene; alcohols such as cyclohexanol, amyl alcohol and ethylene glycol; ketones such as isophorone, cyclohexanone and cyclohexenylcyclohexane; ethers such as butylcellosolve, diethyl ether and methyl ethyl ether; esters such as isopropyl acetate, benzyl acetate and methyl phthalate; amides such as dimethylformamide; and mixtures thereof.

Any of anionic surfactants, nonionic surfactants, cationic surfactants and amphoteric surfactants such as amino acids and betaine can be used.

The herbicidal compositions of the present invention may further contain, if necessary, other herbicidally active components in combination with the azole compounds represented by the formula (Ia), (Ib), (VIa) or (VIb). Examples of the other herbicidally active components may be suitably selected from diphenyl ether herbicides, triazine herbicides, urea herbicides, carbamate herbicides, thiocarbamate herbicides, acid anilide herbicides, pyrazole herbicides, phosphoric acid herbicides, sulfonylurea herbicides and oxadiazone herbicides. The herbicidal compositions of the present invention may further contain insecticides, antibiotics, plant-growth regulators and fertilizers, if necessary.

The herbicidal compositions of the present invention are applied to weeds before and/or after germination or their environments. The modes of application vary depending on the type of the cultivated plants and the environmental factors. For example, the herbicidal compositions may be applied by spraying, scattering, sprinkling or irrigating.

An application rate of the azole compounds of the present invention is determined by a number of factors such as formulation selected, mode of application, amount and type of weed species, growing conditions, etc. In general, the application rate is 0.001 to 10 kg/ha, preferably 0.01 to 5 kg/ha. One skilled in the art can easily determine the application rate necessary for the desired level of weed control.

The herbicidal compounds of the present invention are effective for controlling weeds in useful cultivated plants such as Gramineous crops such as rice, wheat, barley, corn, oat and sorghum; broad-leaved crops such as soy bean, cotton, beet, sunflower and rapeseed; fruit trees; vegetables such as fruit vegetables, root vegetables and leaf vegetables; and turf.

The herbicides of the present invention are effective for controlling paddy weeds and cropland weeds. Paddy weeds include Alismataceous weeds such as *Alisma canaliculatum, Sagittaria trifolia* and *Sagittaria pygmaea*; Cyperaceous weeds such as *Cyperus difformis, Cyperus serotinus, Scirpus juncoides* and *Eleocharis kuroguwai*; Scrophulariaceous weeds such as *Lindernia pyxidaria*; Pontenderiaceous weeds such as *Monochoria vaginalis*; Potamogetonaceous weeds such as *Potamogeton distinctus*; Lythraceous weeds such as *Rotala indica*; and Gramineous weeds such as *Echinochloa crug-galli.*

Cropland weeds include broad-leaved weeds and narrow-leaved weeds. Broad-leaved weeds may be Solanaceous weeds such as *Solanum nigrum* and *Datura stramonium*; Malvaceous weeds such as *Abutilon theophrasti* and *Sida spinosa*; Convolvulaceous weeds such as *Ipomoea purpurea*; Amaranthaceous weeds such as *Amaranthus lividus*; Composite weeds such as *Xanthium strumarium, Ambrosia artemisifolia, Galinsoga ciliata, Cirsium arvense, Senecio vulgaris* and *Erigeron annus*; Brasicaceous weeds such as *Rorippa indica, Sinapis arvensis* and *Capsella bursa-pastoris*; Polygonaceous weeds such as *Polygonum bulumei* and *Polygonum convolvulus*; Portulacaceous weeds such as *Portulaca oleracea*; Chenopodiaceous weeds such as *Chenopodium alubum, Chenopodium ficiolium* and *Kochia scoparia*; Caryophyllaceous weeds such as *Stellaria media*; Scrophulariaceous weeds such as *Veronica persica*; Commelinaceous weeds such as *Commelina communis*; Euphorbiaceous weeds such as *Lamium amplexicaule, Euphorbia supina* and *Euphorbia maculata*; Rubiaceous weeds such as *Galium spurium, Galium aparine* and *Rubia akane*; Violaceous weeds such as *Viola arvensis*; and Leguminous weeds such as *Sesbania exaltata* and *Cassia obtusifolia.* Narrow-leaved weeds may be Graminaceous weeds such as *Sorghum bicolor, Panicum dichotomiflorum, Sorghum haepense, Echinochloa crus-galli, Digitaria adscendens, Avena fatua, Eleusine indica, Setaria viridis* and *Alopecurus aequalis*; and Cyperaceous weeds such as *Cyperus rotundus* and *Cyperus esculentus.*

The present invention will be described more specifically with reference to the following examples.

Example 1

[1] Synthesis of 4-chloro-5-oxycarbonyl-2,3-dihydrobenzothiophene 1,1-dioxide

(1) Synthesis of ethyl 4-carboxymethylsulfenyl-2-chlorobenzoate

A mixture of 10.0 g of ethyl 2,4-dichlorobenzoate, 9.44 g of potassium carbonate, 50 ml of dimethylformamide and 3.8 ml of mercaptoacetic acid was heated at 80°C for 4 hours.

Then, the resultant reaction mixture was poured into ice water and extracted with ethyl acetate. The extract was dried over sodium sulfate and filtrated. By concentrating the filtrate, 12.3 g of a crude product were obtained.

(2) Synthesis of 4-chloro-5-ethoxycarbonyl-3-oxo-2,3-dihydrobenzothiophene

A mixture of 12.3 g of ethyl 4-carboxymethylsulfenyl-2-chlorobenzoate obtained in (1), 36 ml of 1,2-dichloroethane and 3.9 ml of thionyl chloride was heated at reflux for one hour.

The reaction mixture was concentrated to obtain acid chloride, which was then dissolved in 36 ml of dichloromethane. The resultant solution was added dropwise to a solution, prepared in advance, of 14.3 g (107 mmol) of aluminum chloride in 150 ml of dichloromethane over one hour at freezing temperature, and the reaction was further continued at room temperature for two hours. The resultant reaction product was poured into ice water and extracted with dichloromethane. The extract was dried over sodium sulfate, followed by filtration and concentration to give 12.3 g of a crude product as dark brown oil. After purification of the crude product by column chromatography, 5.3 g (yield: 46%) of the title compound were obtained as brown oil.

(3) Synthesis of 4-chloro-5-ethoxycarbonyl-3-hydroxy-2,3-dihydrobenzothiophene

A solution of 5.3 g of 4-chloro-5-ethoxycarbonyl-3-oxo-2,3-dihydrobenzothiophene obtained in (2) in 25 ml of dichloromethane and 25 ml of ethanol was cooled in ice bath. To the solution, 0.26 g of sodium boron hydride was added and the resultant mixture was kept standing overnight. Then, the reaction solution was poured into ice water and extracted with dichloromethane. The extract was dried over sodium sulfate, filtrated and concentrated to give 5.3 g (yield: 98%) of the title compound.

(4) Synthesis of 4-chloro-5-ethoxycarbonylbenzothiophene

4-Chloro-5-ethoxycarbonyl-3-hydroxy-2, 3-dihydrobenzothiophene obtained in (3) (5.3 g) was azeotropically dehydrated by heating with 50 ml of toluene and 0.2 g of p-toluenesulfonic acid for one hour. The resultant solution was diluted with toluene, washed with saturated solution of sodium hydrogencarbonate, dried over sodium sulfate, filtered and concentrated to obtain 4.6 g (yield: 95%) of the title compound as brown oil.

(5) Synthesis of 4-chloro-5-ethoxycarbonylbenzothiophene 1,1-dioxide

A mixture of 4.6 g of 4-chloro-5-ethoxycarbonylbenzothiophene obtained in (4), 30 ml of acetic acid and 5.4 ml of 30% by weight aqueous hydrogen peroxide was heated at 80°C for two hours under stirring. After cooling to room temperature, the reaction solution was diluted with water and filtered to collect solid product, which was dried and purified by column chromatography to obtain 3.7 g (yield: 95%) of the title compound as colorless crystals.

(6) Synthesis of 4-chloro-5-ethoxycarbonyl-2,3-dihydrobenzothiophene 1,1-dioxide

4-Chloro-5-ethoxycarbonylbenzothiophene-1,1-dioxide (3.7 g) obtained in (5) was hydrogenated in 40 ml of tetrahydrofuran in the presence of 5% palladium/carbon in hydrogen gas atmosphere at room temperature and atmospheric pressure for 8 hours. The reaction mixture was filtered and concentrated to obtain 3.44 g (yield: 91%) of the title compound as pale yellow oil.

(7) Synthesis of 4-chloro-5-oxycarbonyl-2,3-dihydrobenzothiophene 1,1-dioxide

To a solution of 3.44 g of 4-chloro-5-ethoxycarbonyl-2,3-dihydrobenzothiophene 1,1-dioxide obtained in (6) in 35 ml of ethanol, were added 5 ml of 20% by weight aqueous solution of sodium hydroxide and the resultant mixture was kept standing overnight.

After concentrating, the mixture was made acidic with 5% by weight hydrochloric acid, and the precipitates were collected by filtration and dried to obtain 2.6 g (yield: 84%) of the title compound as colorless crystals.

$^1$H-NMR spectrum (acetone-d$^6$, TMS) of the colorless crystals showed absorption peaks at 3.4-3.8 (m, 4H), 7.85 (1H, d) and 8.06 (1H, d). The infrared spectrum showed absorption peaks at 3080 cm$^{-1}$, 3010 cm$^{-1}$, 1690 cm$^{-1}$, 1410 cm$^{-1}$, 1400 cm$^{-1}$, 1310 cm$^{-1}$, 1290 cm$^{-1}$, 1250 cm$^{-1}$, 1190 cm$^{-1}$ and 1130 cm$^{-1}$. From these results, the obtained crystals were confirmed to be 4-chloro-5-oxycarbonyl-2,3-dihydrobenzothiophene 1,1-dioxide melting at 232 to 233°C.

[2] Synthesis of 4-chloro-5-(1,3-dioxocyclohex-2-yl)carbonyl-2,3-dihydrobenzothiophene 1,1-dioxide

A mixture of 1.0 g of 4-chloro-5-oxycarbonyl-2,3-dihydrobenzothiophene 1,1-dioxide obtained in [1] and 4 ml suspension of 0.54 g of thionyl chloride in 1,2-dichloroethane was heated for one hour at reflux, and thereafter, the solvent was removed by evaporation at reduced pressure to obtain acid chloride. The acid chloride and 0.47 g of

1,3-cyclohexanedione were dissolved in 10 ml of acetonitrile as the solvent to prepare a solution, to which a solution of 0.82 g of triethylamine in 5 ml of acetonitrile was added dropwise.

Then, after stirred at room temperature for two hours, the mixture was treated with 0.01 g of acetone cyanohydrin and stirred at room temperature for 20 hours.

The reaction mixture was diluted with ethyl acetate and extracted with a saturated aqueous solution of sodium carbonate. The aqueous layer was adjusted to pH 1 with 10% hydrochloric acid and extracted with ethyl acetate. The combined organic layer was washed with water and a saturated aqueous solution of sodium chloride and dried over anhydrous sodium sulfate. The solvent was removed from the reaction mixture by evaporation at reduced pressure to obtain 1.24 g (yield: 90%) of product.

$^1$H-NMR spectrum of the product showed absorption peaks at 1.9-2.3 (m, 2H), 2.3-2.7 (m, 2H), 2.6 to 3.0 (m, 2H), 3.1 to 3.8 (m, 4H), 7.32 (1H, d) and 7.69 (1H, d). Infrared spectrum showed absorption peaks at 1675 cm$^{-1}$, 1575 cm$^{-1}$, 1550 cm$^{-1}$, 1400 cm$^{-1}$, 1295 cm$^{-1}$ and 1125 cm$^{-1}$. From these results, the product was confirmed to be 4-chloro-5-(1,3-dioxocylohexa-2-yl)carbonyl-2,3-dihydrobenzothiophene 1,1-dioxide melting at 156.0-159.7°C.

[3] Synthesis of 4-chloro-5-(3-chloro-1-oxo-2-cyclohexen-2-yl)carbonyl-2,3-dihydrobenzothiophene 1,1 dioxide

4-Chloro-5-(1,3-dioxocylohex-2-yl)carbonyl-2,3-dihydrobenzothiophene 1,1-dioxide (1.00 g) obtained in [2] was dissolved in 5 ml of 1,2-dichloroethane as the solvent. The solution was added with 0.36 g of oxalyl chloride and 0.01 g of dimethylformamide at room temperature and heated at reflux for one hour.

The solvent was removed by evaporation at reduced pressure to obtain a crude product, which was then purified by column chromatography to obtain 0.99 g (yield: 94%) of product.

$^1$H-NMR spectrum of the product showed absorption peaks at 2.0-2.4 (m, 2H), 2.4-2.7 (m, 2H), 2.8-3.0 (m, 2H), 3.3-3.7 (m, 4H), 7.70 (1H, d) and 7.82 (1H, d). Infrared spectrum showed absorption peaks at 1683 cm$^{-1}$, 1605 cm$^{-1}$, 1390 cm$^{-1}$, 1300 cm$^{-1}$, 1175 cm$^{-1}$ and 1125 cm$^{-1}$. From these results, the obtained product was confirmed to be 4-chloro-5-(3-chloro-1-oxo-2-cylohexen-2-yl)carbonyl-2,3-dihydrobenzothiophen e 1,1-dioxide melting at 83.8-85.4°C.

[4] Synthesis of 4-chloro-5-[1-oxo-3-(1-pyrazolyl)-2-cyclohexen-2-yl]carbonyl-2,3-dihydrobenzothiophene 1,1-dioxide

Into a suspension of 0.11 g of sodium hydride in 3 ml of tetrahydrofuran, was added 0.19 g of pyrazole at 0°C and the resultant mixture was stirred for 30 minutes.

To the solution, at 0°C, was added a solution of 0.50 g of 4-chloro-5-(3-chloro-1-oxo-2-cyclohexen-2-yl)carbonyl-2,3-dihydrobenzothiophe ne 1,1-dioxide obtained in [3] in 2 ml of tetrahydrofuran as the reaction solvent, and the resultant mixture was stirred at room temperature for 5 hours.

After the reaction was completed, the reaction solution was added with 5% by weight aqueous solution of hydrochloric acid and extracted with ethyl acetate. The extract was washed with saturated aqueous solution of sodium chloride and dried over anhydrous sodium sulfate. The crude product obtained by removing the solvent by evaporation at reduced pressure was purified by column chromatography to obtain 0.45 g (yield: 83%) of product.

[4'] Synthesis of 4-chloro-5-[1-oxo-3-(1-pyrazolyl)-2-cyclohexen-2-yl]carbonyl-2,3-dihydrobenzothiophene 1,1-dioxide

The title compound was synthesized by a process different from the process of [4].

4-Chloro-5-(3-chloro-1-oxo-2-cyclohexen-2-yl)carbonyl-2,3-dihydrobenzot hiophene 1,1-dioxide (0.50 g) obtained in [3] and 0.19 g of pyrazole were dissolved in 5 ml of methylene chloride as the solvent. The solution was added with triethylamine as the catalyst at room temperature and the mixture was stirred at room temperature for 5 hours to allow the reaction to proceed. After the reaction was completed, the solvent was removed by evaporation at reduced pressure to obtain a crude product, which was then purified by column chromatography to obtain 0.45 g (yield: 83%) of product.

The results of $^1$H-NMR and IR analysis, the chemical structure and the melting point of the product obtained in [4] and [4'] are shown in Table 1. From the results, the product obtained above was confirmed to be 4-chloro-5-[1-oxo-3-(1-pyrazolyl)-2-cyclohexen-2-yl]carbonyl-2,3-dihydrobenzoth iophene 1,1-dioxide (Compound No. 1).

Example 2

Synthesis of 4-chloro-5-[1-oxo-3-(3-methyl-1-pyrazolyl)-2-cyclohexen-2-yl]carbonyl-2,3-dihydrobenzothiophene 1,1-dioxide (Compound No. 2)

The title compound was produced in the same manner as in [4] of Example 1 except that 3-methylpyrazole was used in place of pyrazole.

$^1$H-NMR data, IR data, the chemical structure and the melting point of the title compound are shown in Table 1.

Example 3

Synthesis of 4-chloro-5-[1-oxo-3-(4-methyl-1-pyrazolyl)-2-cyclohexen-2-yl]carbonyl-2,3-dihydrobenzothiophene 1,1-dioxide (Compound No. 3)

The title compound was produced in the same manner as in [4] of Example 1 except that 4-methylpyrazole was used in place of pyrazole.

$^1$H-NMR data, IR data, the chemical structure and the melting point of the title compound are shown in Table 1.

Example 4

Synthesis of 4-chloro-5-[1-oxo-3-(4-bromo-1-pyrazolyl)-2-cyclohexen-2-yl]carbonyl-2, 3-dihydrobenzothiophene 1,1-dioxide (Compound No. 4)

The title compound was produced in the same manner as in [4] of Example 1 except that 4-bromopyrazole was used in place of pyrazole.

$^1$H-NMR data, IR data, the chemical structure and the melting point of the title compound are shown in Table 1.

Example 5

Synthesis of 4-chloro-5-[1-oxo-3-(3-methyl-4-bromo-1-pyrazolyl)-2-cyclohexen-2-yl]carbonyl-2,3-dihydrobenzothiophene 1,1-dioxide

(Compound No. 5)

The title compound was produced in the same manner as in [4] of Example 1 except that 3-methyl-4-bromopyrazole was used in place of pyrazole.

$^1$H-NMR data, IR data, the chemical structure and the melting point of the title compound are shown in Table 1.

Example 6

Synthesis of 4-chloro-5-[1-oxo-3-(3-methyl-4-phenyl-1-pyrazolyl)-2-cyclohexen-2-yl]carbonyl-2,3-dihydrobenzothiophene 1,1-dioxide

(Compound No. 6)

The title compound was produced in the same manner as in [4] of Example 1 except that 3-methyl-4-phenylpyrazole was used in place of pyrazole.

$^1$H-NMR data, IR data, the chemical structure and the melting point of the title compound are shown in Table 1.

Example 7

Synthesis of 4-chloro-5-[1-oxo-3-(1,2,5-triazol-1-yl)-2-cyclohexen-2-yl]carbonyl-2,3-dihydrobenzothiophene 1,1-dioxide (Compound No. 7)

The title compound was produced in the same manner as in [4] of Example 1 except that 1,2,5-triazole was used in place of pyrazole.

$^1$H-NMR data, IR data, the chemical structure and the melting point of the title compound are shown in Table 1.

Example 8

Synthesis of 4-chloro-5-[3-(1,2,4-triazol-1-yl)-2-cyclohexen-1-oxo-2-yl]carbonyl-2,3-dihydrobenzothiophene 1,1-dioxide (Compound No. 8)

The title compound was produced in the same manner as in [4] of Example 1 except that 1,2,4-triazole was used in place of pyrazole.

$^1$H-NMR data, IR data, the chemical structure and the melting point of the title compound are shown in Table 1.

Example 9

Synthesis of 4-chloro-5-[3-(1-indazolyl)-2-cyclohexen-1-one-2-yl]carbonyl-2,3-dihydrobenzothiophene 1,1-dioxide (Compound No. 9)

The title compound was produced in the same manner as in [4] of Example 1 except that indazole was used in place of pyrazole used in [4] of Example 1.

$^1$H-NMR data, IR data, the chemical structure and the melting point of the title compound are shown in Table 1.

Example 10

Synthesis of 4-methyl-5-[1-oxo-3-(1-pyrazolyl)-2-cyclohexen-2-yl]carbonyl-2,3-dihydrobenzothiophene 1,1-dioxide (Compound No. 10)

The title compound was produced in the same manner as in [4] of Example 1 except that

4-methyl-5-(3-chloro-1-oxo-2-cyclohexen-2-yl)carbonyl-2,3-dihydrobenzothiophe ne 1,1-dioxide was used in place of
4-chloro-5-(3-chloro-1-oxo-2-cyclohexen-2-yl)carbonyl-2,3-dihydrobenzothiophe ne 1,1-dioxide.

$^1$H-NMR data, IR data, the chemical structure and the melting point of the title compound are shown in Table 1.

Example 11

Synthesis of 5-chloro-6-[1-oxo-3-(1-pyrazolyl)-2-cyclohexen-2-yl]carbonylthiochroman 1,1-dioxide (Compound No. 11)

The title compound was produced in the same manner as in [4] of Example 1 except that

5-chloro-5-(3-chloro-1-oxo-2-cyclohexen-2-yl)carbonylthiochroman 1,1-dioxide was used in place of
4-chloro-5-(3-chloro-1-oxo-2-cyclohexen-2-yl)carbonyl-2, 3-dihydrobenzothiophe ne 1,1-dioxide.

$^1$H-NMR data, IR data, the chemical structure and the melting point of the title compound are shown in Table 1.

Example 12

Synthesis of 4-cyano-5-[1-oxo-3-(1-pyrazolyl)-2-cyclohexen-2-yl]carbonyl-2,3-dihydrobenzothiophene 1,1-dioxide (Compound No. 12)

The title compound was produced in the same manner as in [4] of Example 1 except that

4-cyano-5-(3-chloro-1-oxo-2-cyclohexen-2-yl)carbonyl-2,3-dihydrobenzothiophen e 1,1-dioxide was used in place of
4-chloro-5-(3-chloro-1-oxo-2-cyclohexen-2-yl)carbonyl-2,3-dihydrobenzothiophe ne 1,1-dioxide.

$^1$H-NMR data, IR data, the chemical structure and the melting point of the title compound are shown in Table 1.

Example 13

Synthesis of 4-chloro-3-methoxy-5-[1-oxo-3-(1-pyrazolyl)-2-cyclohexen-2-yl]carbonyl-2,3-dihydrobenzothiophene 1,1-dioxide (Compound No. 13)

The title compound was produced in the same manner as in [4] of Example 1 except that

4-chloro-3-methoxy-5-(3-chloro-1-oxo-2-cyclohexen-2-yl)carbonyl-2,3-dihydrobe nzothiophene 1,1-dioxide was used in place of
4-chloro-5-(3-chloro-1-oxo-2-cyclohexen-2-yl)carbonyl-2,3-dihydrobenzothiophe ne 1,1-dioxide.

$^1$H-NMR data, IR data, the chemical structure and the melting point of the title compound are shown in Table 1.

Example 14

Synthesis of 4-chloro-7-[1-oxo-3-(1-pyrazolyl)-2-cyclohexen-2-yl]carbonyl-2,3-dihydrobenzothiophene 1,1-dioxide (Compound No. 14)

The title compound was produced in the same manner as in [4] of Example 1 except that

4-chloro-7-(3-chloro-1-oxo-2-cyclohexen-2-yl)carbonyl-2,3-dihydrobenzothiophe ne 1,1-dioxide was used in place of
4-chloro-5-(3-chloro-1-oxo-2-cyclohexen-2-yl)carbonyl-2, 3-dihydrobenzothiophe ne 1,1-dioxide.

$^1$H-NMR data, IR data, the chemical structure and the melting point of the title compound are shown in Table 1.

Example 15

Synthesis of 4-chloro-5-{ 1-oxo-3-[1-(4,5-dihydropyrazolyl)]-2-cyclohexen-2-yl}carbonyl-2,3-dihydrobenzothiophene 1,1-dioxide (Compound No. 15)

The title compound was produced in the same manner as in [4] of Example 1 except that 4,5-dihydropyrazole was used in place of pyrazole.
$^1$H-NMR data, IR data, the chemical structure and the melting point of the title compound are shown in Table 1.

Table 1

| Compound No. | Chemical Structure | NMR ppm($CDCl_3$, TMS standard) | IR $cm^{-1}$ | Melting Point (°C) |
|---|---|---|---|---|
| 1 | 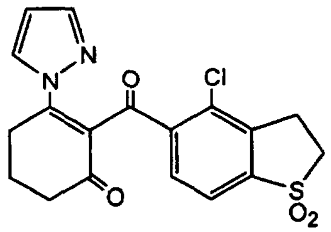 | 2.2-2.5(2H,m)<br>2.5-2.8(2H,m)<br>2.9-3.2(2H,m)<br>3.3-3.6(4H,m)<br>6.44(1H,dd)<br>7.52(1H,d)<br>7.63(1H,d)<br>7.77(1H,d)<br>7.97(1H,d) | 1660<br>1615<br>1400<br>1380<br>1180<br>1120 | 209.1-213.7 |
| 2 | 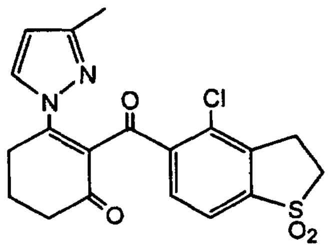 | 2.06(3H,s)<br>2.2-2.4(2H,m)<br>2.5-2.7(2H,m)<br>2.9-3.1(2H,m)<br>3.3-3.7(4H,m)<br>6.20(1H,d)<br>7.62(1H,d)<br>7.64(1H,d)<br>7.96(1H,d) | 1690<br>1615<br>1545<br>1410<br>1305<br>1185<br>1135 | 213.1-213.2 |
| 3 | 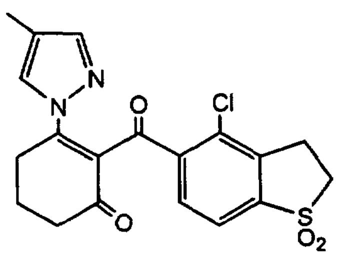 | 2.05(3H,s)<br>2.2-2.4(2H,m)<br>2.5-2.7(2H,m)<br>2.9-3.1(2H,m)<br>3.3-3.7(4H,m)<br>7.34(1H,s)<br>7.51(1H,s)<br>7.61(1H,d)<br>7.94(1H,d) | 1690<br>1655<br>1615<br>1415<br>1300<br>1180<br>1135 | 106.2-109.0 |
| 4 | 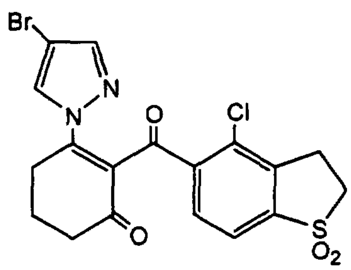 | 2.2-2.5(2H,m)<br>2.5-2.8(2H,m)<br>2.9-3.1(2H,m)<br>3.2-3.8(4H,m)<br>7.47(1H,s)<br>7.64(1H,d)<br>7.80(1H,s)<br>7.95(1H,s) | 1695<br>1620<br>1410<br>1295<br>1185<br>1140<br>1120 | 166.0-167.2 |

| | | | | |
|---|---|---|---|---|
| 5 | 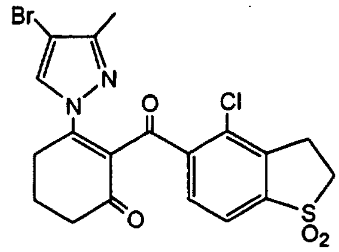 | 2.04(3H,s)<br>2.2-2.4(2H,m)<br>2.5-2.7(2H,m)<br>2.9-3.1(2H,m)<br>3.3-3.7(4H,m)<br>7.64(1H,d)<br>7.71(1H,s)<br>7.96(1H,d) | 1695<br>1650<br>1615<br>1410<br>1300<br>1140<br>1120 | 212.7-215.9 |
| 6 | 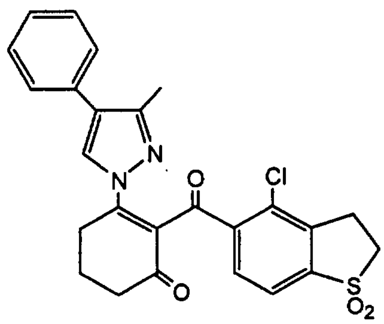 | 2.15(3H,s)<br>2.2-2.5(2H,m)<br>2.5-2.7(2H,m)<br>2.9-3.2(2H,m)<br>3.3-3.7(4H,m)<br>7.2-7.4(5H,m)<br>7.64(1H,d)<br>7.76(1H,s)<br>8.01(1H,d) | 1695<br>1645<br>1605<br>1410<br>1295<br>1190<br>1130 | 229.8-232.1 |
| 7 | 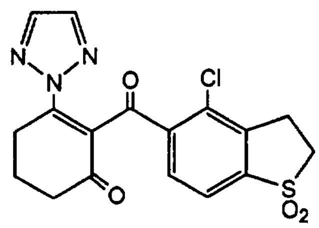 | 2.2-2.5(2H,m)<br>2.5-2.8(2H,m)<br>3.2-3.3(6H,m)<br>7.65(1H,d)<br>7.75(2H,s)<br>8.02(1H,d) | 1695<br>1660<br>1625<br>1390<br>1310<br>1270<br>1135 | 88.3-92.5 |
| 8 | 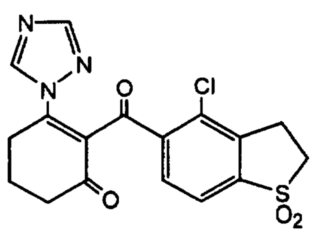 | 2.2-2.5(2H,m)<br>2.5-2.8(2H,m)<br>3.0-3.2(2H,m)<br>3.3-3.7(4H,m)<br>7.67(1H,d)<br>7.89(1H,s)<br>7.99(1H,d)<br>8.50(1H,s) | 1685<br>1655<br>1510<br>1410<br>1305<br>1185<br>1135 | 90.6-101.6 |
| 9 | 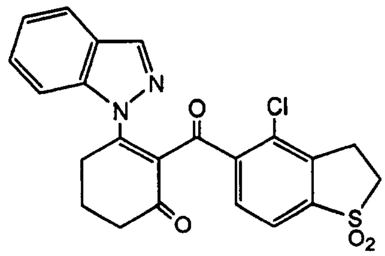 | 2.3-2.5(2H,m)<br>2.6-2.8(2H,m)<br>3.1-3.7(6H,m)<br>6.9-7.6(4H,m)<br>7.59(1H,d)<br>7.97(1H,d)<br>8.23(1H,d) | 1690<br>1655<br>1615<br>1395<br>1300<br>1185<br>1135 | 103.9-109.3 |
| 10 | 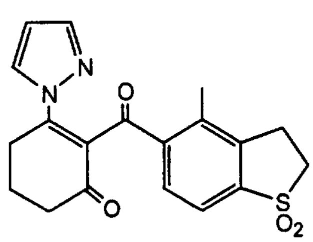 | 2.2-2.4(2H,m)<br>2.5-2.7(2H,m)<br>2.65(3H,s)<br>3.0-3.2(2H,m)<br>3.3-3.6(4H,m)<br>6.42(1H,dd)<br>7.47(1H,d)<br>7.51(1H,d)<br>7.62(1H,d)<br>7.76(1H,d) | 1690<br>1655<br>1615<br>1375<br>1290<br>1185<br>1120<br>760 | 265.6-288.2 |

| | | | | |
|---|---|---|---|---|
| 11 | 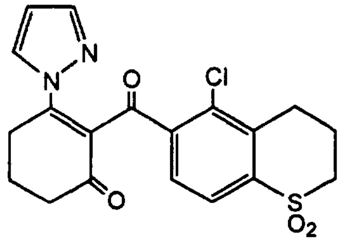 | 2.2-3.5(12H,m)<br>6.42(1H,dd)<br>7.53(1H,d)<br>7.75(1H,d)<br>7.86(2H,s) | 1695<br>1615<br>1375<br>1295<br>1190<br>1125 | Syrup |
| 12 | 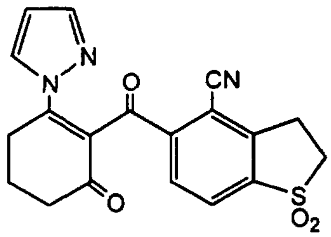 | 2.2-2.5(2H,m)<br>2.6-2.8(2H,m)<br>3.0-3.2(2H,m)<br>3.60(4H,s)<br>6.45(1H,dd)<br>7.50(1H,d)<br>7.83(1H,d)<br>7.98(1H,d)<br>8.16(1H,d) | 2960<br>1690<br>1615<br>1375<br>1300<br>1185<br>1120 | Syrup |
| 13 | 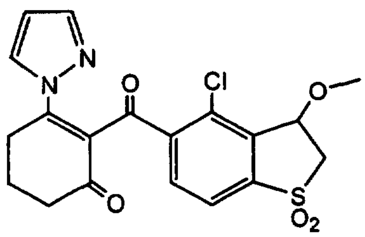 | 2.2-2.4(2H,m)<br>2.5-2.7(2H,m)<br>3.0-3.2(2H,m)<br>3.50(3H,s)<br>3.6-3.7(2H,m)<br>5.24(1H,dd)<br>6.41(1H,dd)<br>7.51(1H,d)<br>7.63(1H,d)<br>7.76(1H,d)<br>8.05(1H,d) | 1695<br>1655<br>1615<br>1395<br>1375<br>1305<br>1200<br>1185<br>1150 | Syrup |
| 14 | 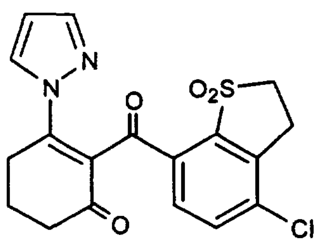 | 2.2-2.5(2H,m)<br>2.5-2.8(2H,m)<br>2.9-3.2(2H,m)<br>3.2-3.7(4H,m)<br>6.34(1H,dd)<br>7.44(1H,d)<br>7.61(1H,d)<br>7.76(1H,d)<br>7.96(1H,d) | 1695<br>1650<br>1620<br>1370<br>1305<br>1260<br>1130<br>760 | 251.1-253.1 |
| 15 | 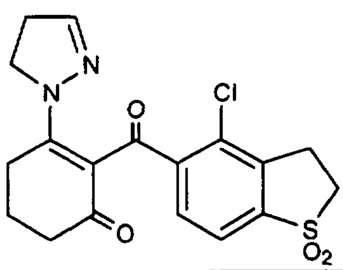 | 1.8-2.2(2H,m)<br>2.2-2.5(2H,m)<br>2.9-3.3(4H,m)<br>3.3-3.8(6H,m)<br>7.32(1H,bs)<br>7.55(1H,d)<br>7.67(1H,d) | 1675<br>1555<br>1430<br>1310<br>1300<br>1175 | 247.8-265.3 |

Example 16

[1] Preparation of Herbicidal Composition

Talc (57 parts by weight), bentonite (40 parts by weight), both as carriers, and a sodium alkylbenzenesulfonate (3 parts by weight) as surfactant were uniformly mixed by pulverization to obtain a carrier for wettable powder formulation. Then, 10 parts by weight of the azole compound obtained in Example 1 and 90 parts by weight of the carrier obtained above were uniformly mixed by pulverization to obtain a herbicidal composition.

[2] Herbicidal Test

(1) Cropland soil treatment

Seeds of six different cropland weed species and two different crop species were sowed on cropland soil in individual Wagner pots of 1/5000 are, and covered with soil. The cropland weeds used were *Echinochloa crus-galli, Setaria viridis*, *Alopecurus myosuroides, Abutilon theophrasti*, *Ambrosia artemisifolia* and *Stellaria media*, and the crops used were corn and wheat. Then, an amount of the herbicidal composition prepared in [1] was suspended in water and the suspension was sprayed uniformly on the surface of the soil.

The pots thus treated and were placed in a green house to allow the seeds to grow. The herbicidal effect and injury to crops were determined 20 days after the treatment with the herbicidal composition. The results are shown in Table 2.

(2) Cropland foliage treatment

Seeds of six different cropland weed species and two different crop species were sowed on cropland soil in individual Wagner pots of 1/5000 are, and covered with soil. The cropland weeds used were *Echinochloa crus-galli, Setaria viridis, Alopecurus myosuroides, Abutilon theophrasti, Ambrosia artemisifolia* and *Stellaria media,* and the crops used were corn and wheat. The pots were then placed in a green house to allow the seeds to grow.

When the plants reached three to four leaf stage, a water suspension containing an amount of the herbicidal composition prepared in [1] was uniformly sprayed on the foliage in a rate of 2,000 liter/ha. The treated plants were then maintained in a green house for vegetation. The herbicidal effect and injury to crops were determined 30 days after the treatment with the herbicidal composition. The results are shown in Table 3.

(3) Irrigation treatment (treatment 3 days after transplantation)

Seeds of *Echinochloa oryzicola* and *Scirpus juncoides* were sowed on the surface of paddy field soil in a Wagner pot of 1/2000. Then, rice seedlings of 2.5 leaf stage were transplanted in the soil.

After supplying water to the pot so as to have a irrigation water depth of 3 cm, the pot was placed in a green house at 20 to 25°C for vegetation.

Three days after the transplantation of rice seedlings, an amount of the herbicidal composition prepared in [1] was added to irrigation water. The herbicidal effect and injury to the paddy rice were determined 30 days after the treatment with the herbicidal composition. The results are shown in Table 4.

(4) Irrigation treatment (treatment 10 days after transplantation)

Seeds of *Echinochloa oryzicola* and *Scirpus juncoides* were sowed on the surface of paddy field soil in a Wagner pot of 1/2000. Then, rice seedlings of 2.5 leaf stage were transplanted in the soil.

The depth of water held in the pot was adjusted to 3 cm and the pot was placed in a green house adjusted at a temperatures of 20 to 25°C. The plants were allowed to grow in the condition for growth of plants.

Three days after the transplantation of rice seedlings, an amount of the herbicidal composition prepared in [1] was added to irrigation water. The herbicidal effect and injury to the paddy rice were determined 30 days after the treatment with the herbicidal composition. The results are shown in Table 4.

The degree of control, the herbicidal effect and the degree of injury to crops in the herbicidal tests (1) to (4) were determined as follows.

(a) Degree of control

The degree of control (%) was calculated from the weights of the above-ground parts of fresh weeds in the

treated area and the untreated area using the following equation:

$$\text{Degree of control (\%)} = [1 - (\text{weight of weeds in treated area}) / (\text{weight of weeds in untreated area})] \times 100$$

(b) Herbicidal effect

The herbicidal effect was determined based on the following ratings:

| [Herbicidal effect] | [Degree of control] |
|---|---|
| 0 | smaller than 5% (almost no effect) |
| 1 | 5% to smaller than 20% |
| 2 | 20% to smaller than 40% |
| 3 | 40% to smaller than 70% |
| 4 | 70% to smaller than 90% |
| 5 | 90% or greater (almost complete control) |

(c) Injury to crops

The injury to crops was evaluated based on the following ratings:

| [Injury to crops] | [Observed injury] |
|---|---|
| 0 | no injury |
| 1 | almost no injury |
| 2 | slight injury |
| 3 | significant injury |
| 4 | marked injury |
| 5 | almost complete death |

Examples 17 to 29

[1] Preparation of herbicidal compositions

Herbicidal compositions were prepared in the same manner as in [1] of Example 16 except that the azole compound was replaced by the azole compounds of Examples 2 to 13 and 15.

[2] Herbicidal tests

The herbicidal tests were conducted in the same manner as in [2] of Example 16 except that the herbicidal composition was replaced by the herbicidal compositions prepared in [1] above.
The results of the herbicidal tests are shown in Tables 2 to 4.

Table 2

| Example No. | Compound No. | Application rate (g/ha) | Herbicidal effect | | | | | | Injury | |
|---|---|---|---|---|---|---|---|---|---|---|
| | | | A* | B* | C* | D* | E* | F* | I* | II* |
| 16 | 1 | 3000 | 5 | 4 | 3 | 5 | 5 | 4 | 0 | 1 |
| 17 | 2 | 3000 | 5 | 4 | 3 | 5 | 5 | 3 | 0 | 1 |
| 18 | 3 | 3000 | 5 | 5 | 3 | 5 | 4 | 4 | 0 | 1 |
| 19 | 4 | 3000 | 5 | 4 | 3 | 5 | 5 | 4 | 0 | 0 |
| 20 | 5 | 3000 | 4 | 3 | 3 | 5 | 5 | 3 | 0 | 0 |
| 21 | 6 | 3000 | 4 | 3 | 2 | 4 | 4 | 3 | 0 | 0 |
| 22 | 7 | 3000 | 5 | 3 | 3 | 5 | 5 | 3 | 0 | 0 |
| 23 | 8 | 3000 | 5 | 4 | 3 | 5 | 5 | 4 | 0 | 1 |
| 24 | 9 | 3000 | 5 | 3 | 3 | 5 | 5 | 3 | 0 | 0 |
| 25 | 10 | 3000 | 5 | 4 | 3 | 5 | 5 | 4 | 0 | 0 |
| 26 | 11 | 3000 | 5 | 4 | 3 | 5 | 5 | 4 | 0 | 0 |
| 27 | 12 | 3000 | 5 | 4 | 3 | 5 | 4 | 3 | 0 | 0 |
| 28 | 13 | 3000 | 5 | 5 | 3 | 5 | 5 | 5 | 0 | 1 |
| 29 | 15 | 3000 | 5 | 4 | 3 | 5 | 5 | 4 | 0 | 1 |

A* is *Echinochloa crus-galli*,
B* is *Setaria viridis*,
C* is *Alopecurus myosuroides*,
D* is *Abutilon theophrasti*,
E* is *Ambrosia artemisifolia*,
F* is *Stellaria media,*
I* is corn and
II* is wheat.

Table 3

| Example No. | Compound No. | Application rate (g/ha) | Herbicidal effect | | | | | | Injury | |
|---|---|---|---|---|---|---|---|---|---|---|
| | | | A* | B* | C* | D* | E* | F* | I* | II* |
| 16 | 1 | 3000 | 5 | 4 | 3 | 5 | 5 | 4 | 0 | 1 |
| 17 | 2 | 3000 | 5 | 3 | 4 | 5 | 5 | 5 | 0 | 2 |
| 18 | 3 | 3000 | 5 | 4 | 3 | 5 | 5 | 4 | 0 | 2 |
| 19 | 4 | 3000 | 5 | 5 | 4 | 5 | 5 | 5 | 0 | 2 |
| 20 | 5 | 3000 | 5 | 3 | 3 | 5 | 5 | 4 | 0 | 1 |
| 21 | 6 | 3000 | 4 | 2 | 2 | 5 | 4 | 3 | 0 | 0 |
| 22 | 7 | 3000 | 5 | 5 | 4 | 5 | 5 | 5 | 0 | 2 |
| 23 | 8 | 3000 | 5 | 5 | 4 | 5 | 5 | 5 | 0 | 1 |
| 24 | 9 | 3000 | 5 | 3 | 3 | 5 | 5 | 4 | 0 | 0 |
| 25 | 10 | 3000 | 5 | 5 | 4 | 5 | 5 | 5 | 0 | 1 |
| 26 | 11 | 3000 | 5 | 5 | 3 | 5 | 5 | 5 | 0 | 1 |
| 27 | 12 | 3000 | 5 | 4 | 3 | 5 | 5 | 4 | 0 | 0 |

A* is *Echinochloa crus-galli*,
B* is *Setaria viridis*,
C* is *Alopecurus myosuroides*,
D* is *Abutilon theophrasti*,
E* is *Ambrosia artemisifolia*,
F* is *Stellaria media,*
I* is corn and
II* is wheat.

Table 3 (continued)

| Example No. | Compound No. | Application rate (g/ha) | Herbicidal effect | | | | | | Injury | |
|---|---|---|---|---|---|---|---|---|---|---|
| | | | A* | B* | C* | D* | E* | F* | I* | II* |
| 28 | 13 | 3000 | 5 | 5 | 4 | 5 | 5 | 5 | 1 | 2 |
| 29 | 15 | 3000 | 5 | 4 | 3 | 5 | 5 | 4 | 0 | 1 |

A* is *Echinochloa crus-galli*,
B* is *Setaria viridis*,
C* is *Alopecurus myosuroides*,
D* is *Abutilon theophrasti*,
E* is *Ambrosia artemisifolia*,
F* is *Stellaria media,*
I* is corn and
II* is wheat.

Table 4

| | | | Treatment 3 days after transplantation | | | Treatment 10 days after transplantation | |
|---|---|---|---|---|---|---|---|
| Example No. | Compound No. | Application rate (g/ha) | Herbicidal effect | | Injury | Herbicidal effect | |
| | | | G* | H* | III* | G* | H* |
| 16 | 1 | 300 | 5 | 5 | 0 | 5 | 5 |
| 17 | 2 | 300 | 5 | 5 | 0 | 5 | 4 |
| 18 | 3 | 300 | 5 | 5 | 0 | 4 | 5 |
| 19 | 4 | 300 | 5 | 5 | 0 | 5 | 5 |
| 20 | 5 | 300 | 5 | 5 | 0 | 4 | 5 |
| 21 | 6 | 300 | 1 | 3 | 0 | 2 | 3 |
| 22 | 7 | 300 | 5 | 5 | 0 | 2 | 5 |
| 23 | 8 | 300 | 5 | 5 | 0 | 5 | 5 |
| 24 | 9 | 300 | 5 | 5 | 0 | 1 | 4 |
| 25 | 10 | 300 | 5 | 5 | 0 | 4 | 4 |
| 26 | 11 | 300 | 5 | 5 | 0 | 5 | 5 |
| 27 | 12 | 300 | 5 | 5 | 0 | 4 | 5 |
| 28 | 13 | 300 | 5 | 4 | 1 | 5 | 3 |
| 29 | 15 | 300 | 5 | 5 | 0 | 5 | 5 |

G* is *Echinochloa oryzicola*,
H* is *Scirpus juncoides* and
III* is paddy rice.

## Industrial Applicability

The azole compounds of the present invention are useful as the effective components of herbicidal compositions and less injurious to cultivated crops such as field crops and paddy rice, and has a broad spectrum of weed control with a low application rate.

## Claims

**1.** An azole compound represented by the following formula (Ia) or (Ib):

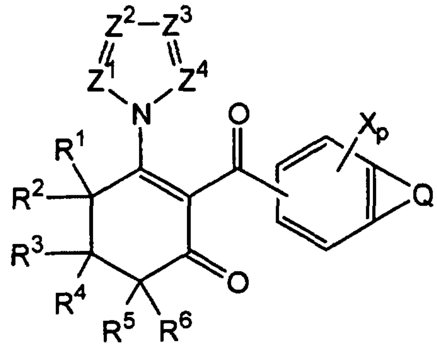

(Ia),

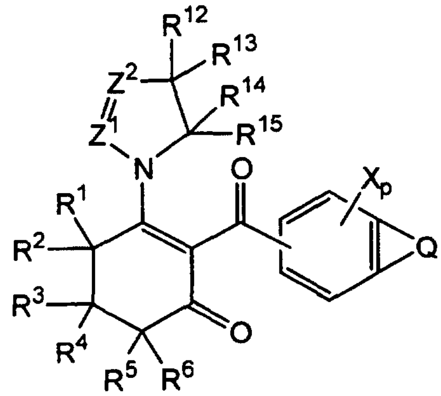

(Ib)

wherein:

Q is a divalent group having 3 to 5 ring-forming atoms which form a five- to seven-membered saturated or unsaturated condensed ring together with two benzene ring carbons to which Q is bonded, one or two of said ring-forming atoms being atom or atoms selected from nitrogen, oxygen and sulfur;
said ring-forming atoms constituting Q may have one or more substituents selected from the group consisting of $C_1$-$C_6$ alkyl, $C_1$-$C_6$ haloalkyl, $C_1$-$C_6$ alkoxy, $C_1$-$C_6$ haloalkoxy, hydroxyl, mercapto, oxo, thioxo, hydroxyimino, $C_1$-$C_6$ alkoxyimino, hydrazono, $C_1$-$C_6$ monoalkylhydrazono and $C_1$-$C_6$ dialkylhydrazono;
the ring-forming atom constituting Q or a pair of adjacent ring-forming atoms may be substituted by a divalent substituent selected from the group consisting of ethylenedioxy, ethylenedithio, propylenedioxy and propylenedithio, said divalent substituent being optionally substituted by halogen or $C_1$-$C_6$ alkyl;
X is halogen , $C_1$-$C_6$ alkyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylsulfinyl, $C_1$-$C_6$ alkylsulfonyl, amino, $C_1$-$C_6$ monoalkylamino, $C_1$-$C_6$ dialkylamino, cyano or nitro;
p is 1 or 2;
$R^1$ to $R^6$ are each hydrogen, halogen, $C_1$-$C_6$ alkyl or $C_1$-$C_6$ haloalkyl;
$R^{12}$ to $R^{15}$ are each hydrogen, halogen, $C_1$-$C_6$ alkyl, $C_1$-$C_6$ haloalkyl, nitro or phenyl which may be substituted; and
$Z^1$ to $Z^4$ are each nitrogen or $CR^7$ wherein $R^7$ is hydrogen, halogen, $C_1$-$C_6$ alkyl, $C_1$-$C_6$ haloalkyl, nitro, phenyl which may be substituted, and a pair of adjacent $CR^7$, if any, may together form a benzene ring which may be substituted by halogen, $C_1$-$C_6$ alkyl, $C_1$-$C_6$ haloalkyl or nitro.

**2.** The azole compound according to Claim 1, wherein $Z^1$ is nitrogen.

**3.** The azole compound according to Claim 1, wherein at least one of two ring-forming atoms boded to the benzene ring is sulfur.

**4.** The azole compound according to Claim 1, wherein $Z^1$ is nitrogen and at least one of two ring-forming atoms bonded to the benzene ring is sulfur.

**5.** A herbicidal composition comprising an effective amount of an azole compound described in any one of Claims 1 to 4.

**6.** An azole compound represented by the following formula (VIa) or (VIb):

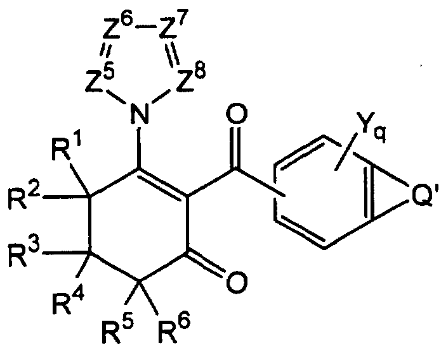

(VIa),

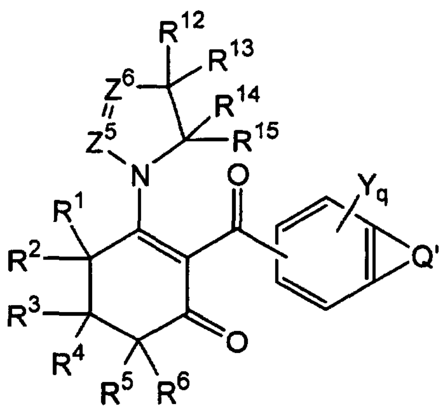

(VIb),

wherein:

$R^1$ to $R^6$ are each hydrogen, halogen, $C_1$-$C_6$ alkyl or $C_1$-$C_6$ haloalkyl;
$R^{12}$ to $R^{15}$ are each hydrogen, halogen, $C_1$-$C_6$ alkyl, $C_1$-$C_6$ haloalkyl, nitro or phenyl which may be substituted;
$Z^5$ to $Z^8$ are each nitrogen or $CR^{11}$ wherein $R^{11}$ is hydrogen, halogen, $C_1$-$C_6$ alkyl, $C_1$-$C_6$ haloalkyl, nitro, cyano or phenyl which may be substituted, and when adjacent $Z^i$ and $Z^{i+1}$ wherein i is 5, 6 or 7 are both $CR^{11}$, the substituents on the carbon atoms may together form a benzene ring which may be substituted by halogen, $C_1$-$C_6$ alkyl, $C_1$-$C_6$ haloalkyl or nitro;

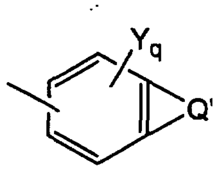

is 5 to 7-membered, saturated or unsaturated condensed ring represented by the following formula:

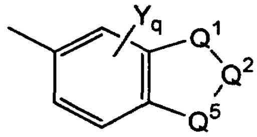 , 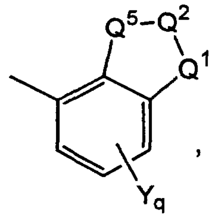 , 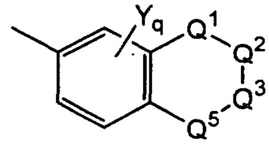 ,

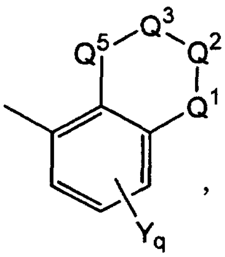 , 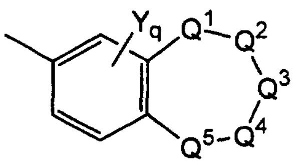 or 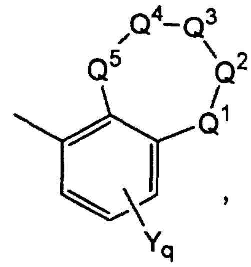 ,

wherein up to two of ring-forming atoms $Q^1$ to $Q^5$ are atoms selected from nitrogen, oxygen and sulfur, with the proviso that $Q^1$ is not sulfur, said ring-forming atoms $Q^1$ to $Q^5$ optionally having one or more substituents selected from the group consisting of $C_1$-$C_6$ alkyl, $C_1$-$C_6$ haloalkyl, $C_1$-$C_6$ alkoxy, $C_1$-$C_6$ haloalkoxy, hydroxyl, mercapto, oxo, thioxo, hydroxyimino, $C_1$-$C_6$ alkoxyimino, hydrazono, $C_1$-$C_6$ monoalkylhydrazono and $C_1$-$C_6$ dialkylhydrazono, and said ring-forming atom of Q' or a pair of adjacent ring-forming atoms being optionally substituted by a divalent substituent selected from the group consisting of ethylenedioxy, ethylenedithio, propylenedioxy and propylenedithio to form a ring structure which may be substituted by halogen or $C_1$-$C_6$ alkyl;
Y is halogen, nitro, amino, cyano, hydroxy, mercapto, $R^8$, $OR^8$, $SR^8$, $SO_2R^8$, $NR^9R^{10}$ or $NHCOR^8$ wherein $R^8$ is linear, branched or cyclic $C_1$-$C_6$ alkyl which may contain an unsaturated bond, linear, branched or cyclic $C_1$-$C_6$ haloalkyl which may contain an unsaturated bond, phenyl which may be substituted or benzyl which may be substituted, and $R^9$ and $R^{10}$ are each hydrogen, linear, branched or cyclic $C_1$-$C_6$ alkyl which may contain an unsaturated bond, linear, branched or cyclic $C_1$-$C_6$ haloalkyl which may contain an unsaturated bond, phenyl which may be substituted or benzyl which may be substituted, said $R^9$ and $R^{10}$ being optionally bonded to each other to form a ring structure; and
q is 0, 1 or 2.

**7.** The azole compound according to claim 6, wherein said ring-forming atom $Q^5$ is sulfur.

**8.** The azole compound according to claim 7, wherein said ring-forming atom $Q^1$ is carbon.

**9.** The azole compound according to claim 7 or 8, wherein said ring-forming atoms $Q^2$ to $Q^4$ are carbons.

**10.** The azole compound according to claim 8 or 9, wherein the substituent on said ring-forming atom $Q^1$ is hydrogen, $C_1$-$C_6$ alkyl, $C_1$-$C_6$ alkoxy, oxo or $C_1$-$C_6$ alkoxyimino.

**11.** The azole compound according to any one of claims 6 to 10, represented by the following formula:

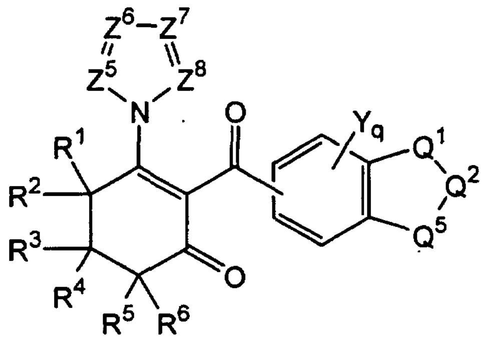
,
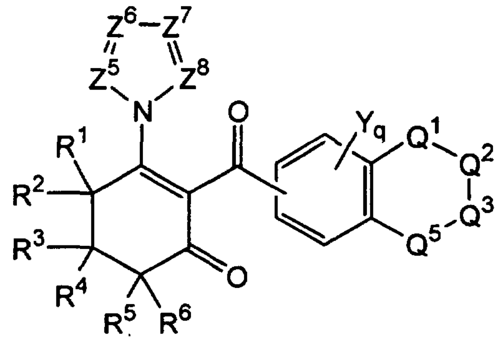
,

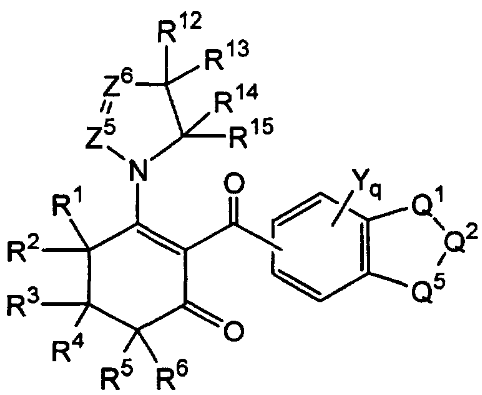
or
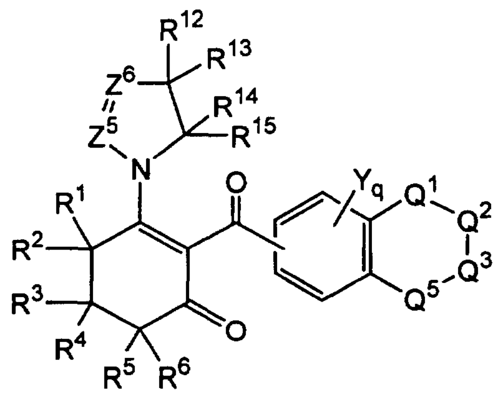
.

**12.** The azole compound according to any one of claims 6 to 11, wherein Y is hydrogen, nitro, cyano, $R^8$, $OR^8$, $SR^8$ or $SO_2R^8$ wherein $R^8$ is as defined in claim 6.

**13.** The azole compound according to any one of claims 6 to 12, wherein $R^1$ to $R^6$ are hydrogens.

**14.** The azole compound according to any one of claims 6 to 13, wherein $Q^1$ is $CH_2$.

**15.** The azole compound according to any one of claims 6 to 13, wherein each of $Q^1$ to $Q^4$ is $CH_2$.

**16.** The azole compound according to any one of claims 6 to 15, wherein $Z^5$ is nitrogen.

**17.** A herbicidal composition comprising, as effective component, an azole compound described in any one of Claims 6 to 16.

**18.** A herbicidal composition for use in paddy rice field, which comprises, as effective component, an azole compound described in any one of Claims 6 to 16.

**INTERNATIONAL SEARCH REPORT**

International application No. PCT/JP00/02154

A. CLASSIFICATION OF SUBJECT MATTER
Int.Cl$^7$ C07D409/08
A01N43/56

According to International Patent Classification (IPC) or to both national classification and IPC

B. FIELDS SEARCHED

Minimum documentation searched (classification system followed by classification symbols)
Int.Cl$^7$ C07D401/00-419/14
A01N43/00-45/02

Documentation searched other than minimum documentation to the extent that such documents are included in the fields searched

Jitsuyo Shinan Koho 1922-1996 Toroku Jitsuyo Shinan Koho 1994-2000
Kokai Jitsuyo Shinan Koho 1971-2000 Jitsuyo Shinan Toroku Koho 1996-2000

Electronic data base consulted during the international search (name of data base and, where practicable, search terms used)
CA(STN)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| EX | WO, 99/57111, A1 (BASF AKTIENGESELLSCHAFT), 11 November, 1999 (11.11.99), Full text; esp. Tables 1-2, & AU, 9940320, A | 1-18 |
| EX | WO, 00/14069, A1 (BASF AKTIENGESELLSCHAFT), 16 March, 2000 (16.03.00), Full text; esp. Table 1 | 1-2,5-6<br>11-18 |
| Y | WO, 97/1550, A (DU PONT), 16 January, 1997 (16.01.97), Full text & EP, 836600, A & JP, 11-509192, A | 1-18 |
| Y | WO, 97/8164, A1 (DU PONT), 06 March, 1997 (06.03.97), Full text & EP, 846112, A | 1-18 |
| Y | WO, 97/9324, A (BASF AKTIENGESELLSCHAFT), 13 March, 1997 (13.03.97), Full text | 1-18 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search: 18 May, 2000 (18.05.00)

Date of mailing of the international search report: 30 May, 2000 (30.05.00)

Name and mailing address of the ISA/ Japanese Patent Office

Authorized officer

Facsimile No.

Telephone No.

Form PCT/ISA/210 (second sheet) (July 1992)

**INTERNATIONAL SEARCH REPORT**

International application No.
PCT/JP00/02154

C (Continuation). DOCUMENTS CONSIDERED TO BE RELEVANT

| Category* | Citation of document, with indication, where appropriate, of the relevant passages | Relevant to claim No. |
|---|---|---|
| | & EP, 847394, A & JP, 10-510846, A | |
| Y | WO, 97/30986, A1 (BASF AKTIENGESELLSCHAFT), 28 August, 1997 (28.08.97), Full text & EP, 888334, A | 1-18 |
| Y | EP, 249813, A1 (STAUFFER CHEMICAL COMPANY), 23 December, 1987 (23.12.87), Full text & US, 4775411, A1 & JP, 62-298563, A | 1-18 |

Form PCT/ISA/210 (continuation of second sheet) (July 1992)